# EUROPÄISCHE PATENTSCHRIFT

(11) **EP 3 563 380 B1**
(45) Veröffentlichungstag und Bekanntmachung des Hinweises auf die Patenterteilung: **03.09.2025**
(21) Anmeldenummer: 17826253.1
(22) Anmeldetag: 29.12.2017
(51) Int. Cl.: G16H 40/63

(54) **VORRICHTUNG ZUM ÜBERTRAGEN VON BETRIEBS- UND MASCHINENDATEN EINES MEDIZINGERÄTS, MEDIZINGERÄT SOWIE VERFAHREN ZUM ÜBERTRAGEN VON BETRIEBS- UND MASCHINENDATEN EINES MEDIZINGERÄTS**
DEVICE FOR TRANSMITTING OPERATING AND MACHINE DATA OF A MEDICAL APPARATUS, MEDICAL APPARATUS, AND METHOD FOR TRANSMITTING OPERATING AND MACHINE DATA OF A MEDICAL APPARATUS
DISPOSITIF DE TRANSMISSION DE DONNÉES DE FONCTIONNEMENT ET DE DONNÉES MACHINE RELATIVES À UN APPAREIL MÉDICAL, APPAREIL MÉDICAL ET PROCÉDÉ POUR TRANSMETTRE DES DONNÉES DE FONCTIONNEMENT ET DES DONNÉES MACHINE RELATIVES À UN APPAREIL MÉDICAL

(30) Priorität: 30.12.2016 DE 102016125951
(43) Veröffentlichungstag der Anmeldung: 06.11.2019
(73) Patentinhaber: Fresenius Medical Care Deutschland GmbH, 61352 Bad Homburg (DE)
(72) Erfinder: STÖHLEIN, Dieter, 97509 Kohlitzheim/Herlheim (DE); SCHÜLLER, Jürgen, 97631 Bad Königshofen (DE); EBERLEIN, Achim, 97424 Schweinfurt (DE)
(74) Vertreter: Nordmeyer, Philipp Werner
(86) Internationale Anmeldenummer: PCT/EP2017/084790
(87) Internationale Veröffentlichungsnummer: WO 2018/122364

(56) Entgegenhaltungen:
- US-A1- 2009 100 132

## Beschreibung

### Technisches Gebiet

Die vorliegende Erfindung betrifft eine Vorrichtung zum Übertragen von Betriebs- und Maschinendaten eines Medizingeräts an eine Auswertevorrichtung, ein Medizingerät sowie ein Verfahren zum Übertragen von Betriebs- und Maschinendaten eines Medizingeräts an eine Auswertevorrichtung. Das Medizingerät ist bevorzugt ein Gerät zur extrakorporalen Blutbehandlung, beispielsweise ein Dialysegerät zur Durchführung einer Hämodialyse, Hämofiltration, Hämodiafiltration und/oder einer anderen Dialysebehandlung. Das Medizingerät kann auch ein Peritonealdialysegerät, ein Akutdialysegerät, ein Leberersatztherapiegerät, oder ein Apharesegerät sein.

Beim Betrieb eines Medizingeräts, beispielsweise bei der Durchführung einer Dialysebehandlung, aber auch in anderen Betriebszuständen des Medizingeräts, kann das Medizingerät Betriebs- und Maschinendaten erzeugen. Anhand der Betriebs- und Maschinendaten können beispielsweise Verschleiß von Komponenten des Medizingeräts und/oder Funktionseinschränkungen von Komponenten ermittelt werden. Für die Ursachenermittlung und -behebung kann die Unterstützung eines Servicetechnikers erforderlich sein. Bei einem Teil der erzeugten Betriebs- und Maschinendaten ist es zudem möglich, dass die Ursache nicht durch einen Servicetechniker vor Ort festgestellt und behoben werden kann, so dass in diesem Fall die Unterstützung beispielsweise der Entwicklungsabteilung des Herstellers des Medizingeräts wünschenswert ist. Hierfür ist es hilfreich, die Betriebs- und Maschinendaten des Medizingeräts, bevorzugt selektiv, aufzuzeichnen beziehungsweise mitzuloggen. Beim Hersteller und insbesondere in dessen Entwicklungsabteilung kann zur Betrachtung und Analyse der Betriebs- und Maschinendaten beispielsweise ein Software-Tool verwendet werden, welches spezielle Visualisierungsmöglichkeiten bietet, um ein Auffinden der Ursachen mit anderen Analysemitteln zu ermöglichen.

### Technischer Hintergrund

Bislang erfolgt die Aufzeichnung der zu betrachtenden und zu analysierenden Betriebs- und Maschinendaten beispielsweise unter Verwendung eines tragbaren Computers beziehungsweise Laptops, der an dem Medizingerät angeschlossen wird. Dieser kann jedoch aus Sicherheitsgründen nicht ohne weiteres während des Behandlungsbetriebs des Medizingeräts, und insbesondere nicht während einer medizinischen Dialysebehandlung, mit diesem verbunden werden. Ferner ist ein speziell für medizinische Anwendungen zugelassenes Netzteil des tragbaren Computers erforderlich. Dies führt dazu, dass beispielsweise Betriebs- und Maschinendaten, die während des Betriebs ermittelt werden, nicht aufgezeichnet werden können.

Die Patentschrift US 8 217 781 B2 beschreibt ein Verfahren zur Erleichterung der Wartung eines Haushaltsgeräts. Die Veröffentlichungsschrift US 2011/185035 A1 beschreibt eine Vorrichtung zur Ursachenanalyse von medizinischen Geräten. Die Veröffentlichungsschriften GB 2 329 983 A, GB 2 225 459 A, EP 0 597 817 A2 und WO 96/10233 A1 beschreiben Datenlogger und/oder demontierbare Datenlogger. Aus der US 2009/100132 A1 ist zudem ein zentraler Kollektor zum Speichern von Daten in Form eines entfernbaren Dongles bekannt.

### Darstellung der Erfindung

Für eine schnelle und vereinfachte Ursachenermittlung und -behebung ist es wünschenswert, Betriebs- und Maschinendaten auch während des Betriebs des Medizingeräts aufzuzeichnen.

Hierbei und im Folgenden wird unter einem Betrieb des Medizingeräts eine Behandlung eines Patienten verstanden. Insbesondere unterscheidet sich der Betrieb von beispielsweise einer Wartung, bei welcher kein Patient behandelt wird und folglich die oben genannten Sicherheitsbetrachtungen nicht relevant sind.

Eine zu lösende Aufgabe der vorliegenden Erfindung besteht entsprechend darin, eine Vorrichtung, ein Medizingerät und ein Verfahren bereitzustellen, welche ein vereinfachtes Übertragen und gegebenenfalls ein weitergehendes Aufzeichnen zur Auswertung von Betriebs- und Maschinendaten eines Medizingeräts erlauben.

Diese Aufgabe wird durch den jeweiligen Gegenstand der unabhängigen Patentansprüche gelöst. Vorteilhafte Weiterbildungen ergeben sich aus den Unteransprüchen, der vorliegenden Beschreibung sowie den Figuren.

Entsprechend wird eine Vorrichtung zum Übertragen von Betriebs- und Maschinendaten eines Medizingeräts, bevorzugt eines Dialysegeräts, an eine Auswertevorrichtung, vorgeschlagen, wobei die Vorrichtung einen Dateneingang zum Empfangen von Betriebs- und Maschinendaten des Medizingeräts, eine programmierbare Speichereinheit, die dazu eingerichtet ist, zumindest einen Teil der empfangenen Betriebs- und Maschinendaten zu speichern, einen Spannungseingang, der dazu eingerichtet ist, die Vorrichtung mit einer von dem Medizingerät bereitgestellten Betriebsspannung zu versorgen und eine Übertragungseinheit, die dazu eingerichtet ist, die gespeicherten Betriebs- und Maschinendaten an die Auswertevorrichtung zu übertragen, umfasst.

Mittels der Vorrichtung lassen sich entsprechend die Betriebs- und Maschinendaten des Medizingeräts auslesen, Zwischenspeichern und dann an die Auswertevorrichtung übergeben. Damit ergibt sich die Möglichkeit der zeitlichen Trennung zwischen dem Empfang und der Speicherung der Betriebs- und Maschinendaten und deren Übergabe an die Auswertevorrichtung.

Mittels der Vorrichtung ergibt sich auch eine elektrische Trennung zwischen dem Medizingerät und der Auswertevorrichtung, so dass eine elektrische Beeinflussung des Medizingeräts durch die Spannungsversorgung und/oder Erdung der Auswertevorrichtung während der Behandlung und/oder die Speicherung und/oder Übertragung und/oder Auswertung der Betriebs- und Maschinendaten durch die Auswertevorrichtung nicht gegeben ist. Entsprechend kann der Empfang und die Speicherung der Betriebs- und Maschinendaten mit der Vorrichtung auch während der eigentlichen medizinischen Behandlung eines Patienten mittels des Medizingeräts durchgeführt werden.

Die Auswertevorrichtung kann ein, bevorzugt tragbarer, Computer sein, insbesondere ein Laptop. Die Auswertevorrichtung kann auch ein mobiles Endgerät sein, beispielsweise ein Smartphone oder ein Tablet. Mittels der Vorrichtung ist es möglich, verschiedene Betriebs- und Maschinendaten des Medizingeräts zu erfassen und weiterzuleiten, ohne dass der Betrieb des Medizingeräts beeinflusst wird und ohne dass die Auswertevorrichtung während der Speicherung der Betriebs- und Maschinendaten bei dem Medizingerät oder mit diesem verbunden verbleiben muss. Das mobile Endgerät, beispielsweise das Smartphone oder Tablet, kann auch in dem Kommunikationspfad zwischen eine Auswertevorrichtung und die Vorrichtung geschaltet sein, sodass die Daten von der Vorrichtung zunächst an das mobile Endgerät und von dort aus an die Auswertevorrichtung übermittelt wird.

Bevorzugt ist der Spannungseingang der Vorrichtung dazu eingerichtet, mit einem Spannungsausgang des Medizingeräts, an dem die Betriebsspannung bereitgestellt wird, verbunden zu werden. Mit anderen Worten: die Vorrichtung wird über das Medizingerät mit Energie versorgt. Die Betriebsspannung kann insbesondere zur Energieversorgung der Speichereinheit und/oder der Übertragungseinheit verwendet werden. Hierzu kann die Vorrichtung weitere elektronische Komponenten zur Aufteilung und/oder Umwandlung der Betriebsspannung enthalten. Es ist möglich, dass sämtliche Komponenten der Vorrichtung ausschließlich mittels der Betriebsspannung mit Energie versorgt werden, wenn die Vorrichtung mit dem Medizingerät verbunden ist. Mit anderen Worten, bei einer Verbindung der Vorrichtung mit dem Medizingerät ist die Vorrichtung mit keiner weiteren Spannungsquelle verbunden. Alternativ oder zusätzlich ist es möglich, dass die Vorrichtung ausschließlich den Spannungseingang als Versorgungsanschluss aufweist und frei von weiteren Versorgungsanschlüssen, die mit einer, insbesondere externen, Spannungsquelle verbunden werden können, ist.

Eine Energieversorgung über das Medizingerät unter Vermeidung weiterer Spannungsquellen kann vor allem bei medizinischen Anwendungen vorteilhaft sein. Insbesondere hat die Vorrichtung aufgrund des Verzichts auf eine externe Spannungsversorgung und die Energieversorgung über das Medizingerät einen viel geringeren Einfluss auf die elektrische und/oder funktionale Sicherheit des zu überwachenden Medizingeräts. Im Gegensatz hierzu benötigt eine alternative Vorrichtung (wie beispielsweise ein Laptop), die mittels einer externen Spannungsversorgung mit Energie versorgt wird, ein speziell für medizinische Anwendungen zugelassenes Netzteil und kann zudem nicht während des Betriebs des Medizingeräts, und insbesondere nicht während einer medizinischen Behandlung, mit dem Medizingerät verbunden sein.

Gemäß zumindest einer Ausführungsform umfasst die Vorrichtung einen Dateneingang zum Empfangen von Betriebs- und Maschinendaten des Medizingeräts. Bevorzugt umfasst die Vorrichtung eine Vielzahl von Dateneingängen. Beispielsweise ist der Dateneingang dazu eingerichtet ist, mit einem Datenausgang des Medizingeräts verbunden zu werden. Bei den Betriebs- und Maschinendaten kann es sich dann um mittels des Datenausgangs übertragene Datensignale handeln. Falls die Vorrichtung eine Vielzahl von Dateneingängen aufweist, können diese dazu eingerichtet sein, jeweils mit einem Datenausgang aus einer Vielzahl von Datenausgängen des Medizingeräts oder mit den Datenausgängen unterschiedlicher Medizingeräte verbunden zu werden. Insbesondere kann jedem Dateneingang der Vorrichtung ein Datenausgang des Medizingeräts eindeutig, bevorzugt eineindeutig, zugeordnet sein.

Gemäß zumindest einer Ausführungsform umfasst die Vorrichtung eine programmierbare Speichereinheit. Die Speichereinheit kann einen Prozessor oder Mikrochip zur Programmierung und ein Speichermedium umfassen. Beispielsweise beinhaltet die Speichereinheit einen Mikrocontroller. Ferner kann die programmierbare Speichereinheit Kommunikationsanschlüsse zum Übertragen, insbesondere Ausgeben und/oder Empfangen der Betriebs- und Maschinendaten umfassen.

Die Speichereinheit ist dazu eingerichtet, zumindest einen Teil der empfangenen Betriebs- und Maschinendaten zu speichern. Bei dem zu speichernden Teil der Betriebs- und Maschinendaten kann es sich beispielsweise um zu überwachende beziehungsweise zu erfassende Betriebs- und Maschinendaten handeln, die beispielsweise für die Auswertevorrichtung von besonderer Bedeutung sind. Mittels des Dateneingangs und der programmierbaren Speichereinheit kann somit beispielsweise ein Datenauslesevorgang durchgeführt werden, bei dem die Betriebs- und Maschinendaten des Medizingeräts mittels des Dateneingangs empfangen werden und ein mittels der Speichereinheit selektierter Teil der Betriebs- und Maschinendaten im Speicher gespeichert wird. Die Selektion kann zum Beispiel mittels einer Konfigurationsvorgabe in der Speichereinheit vorkonfiguriert sein, wobei die Konfigurationsvorgabe beispielsweise von der Auswertevorrichtung und/oder dem mobilen Endgerät vorgegeben und/oder abgeändert werden kann. Zusätzlich ist es möglich, dass die empfangenen Betriebs- und Maschinendaten eine zusätzliche Vorgabe enthalten, anhand derer die zu speichernden Betriebs- und Maschinendaten ermittelt und entsprechend abgespeichert werden.

Anstelle der selektierten Betriebs- und Maschinendaten ist es auch möglich, dass sämtliche Betriebs- und Maschinendaten, die von dem Medizingerät über dessen Datenausgang ausgegeben werden, gespeichert werden.

Das Speichern kann beispielsweise lediglich temporär, das heißt bis zur, insbesondere zeitversetzten beziehungsweise zeitverzögerten, Übertragung der gespeicherten Betriebs- und Maschinendaten an eine Auswertevorrichtung, erfolgen. Auf diese Weise wird eine beispielsweise asynchrone Übertragung der Betriebs- und Maschinendaten ermöglicht, bei der die Auswertevorrichtung nur temporär mit der Übertragungseinheit verbunden ist - beispielsweise wenn sich ein Servicetechniker mit einer entsprechenden Auswertevorrichtung in der Nähe des Medizingeräts befindet oder wenn keine Behandlung mittels des Medizingeräts erfolgt.

Der Dateneingang kann dazu eingerichtet sein, die empfangenen Betriebs- und Maschinendaten, die beispielsweise an dem Datenausgang des Medizingeräts bereitgestellt wurden, an die Speichereinheit weiterzuleiten. Hierzu kann der Dateneingang elektrisch leitend und/oder datentechnisch mit der Speichereinheit, bevorzugt mit dem Prozessor der Speichereinheit, verbunden sein.

Gemäß zumindest einer Ausführungsform umfasst die Vorrichtung eine Übertragungseinheit. Die Übertragungseinheit ist dazu eingerichtet, die gespeicherten Betriebs- und Maschinendaten an die Auswertevorrichtung zu übertragen. Beispielsweise enthält die Übertragungseinheit eine Empfangsantenne zum drahtlosen Senden und/oder Empfangen der gespeicherten Betriebs- und Maschinendaten an die Auswertevorrichtung. Damit ist die Auswertevorrichtung für die Übertragung der Betriebs- und Maschinendaten nicht mit dem Medizingerät verbunden.

Das Übertragen der Betriebs- und Maschinendaten an die Auswertevorrichtung erfolgt mit einer Übertragungsgeschwindigkeit. Die Übertragungsgeschwindigkeit kann sich von einer Empfangsgeschwindigkeit, mit der die Betriebs- und Maschinendaten über den Dateneingang von dem Medizingerät empfangen werden, unterscheiden. Die Empfangsgeschwindigkeit kann die Geschwindigkeit sein, mit der die Betriebs- und Maschinendaten erzeugt und/oder von dem Medizingerät an die Vorrichtung übertragen werden.

Die Übertragungsgeschwindigkeit ist bevorzugt die Maximalgeschwindigkeit des Übertragungsprotokolls, über welches die Übertragungseinheit mit der Auswertevorrichtung verbunden ist. Da die Betriebs- und Maschinendaten in der Speichereinheit konsolidiert werden, können sie bei Bestehen einer Verbindung zwischen der Übertragungseinheit und der Auswertevorrichtung gesammelt und mit der Übertragungsgeschwindigkeit übergeben werden.

Bei der hier beschriebenen Vorrichtung wird insbesondere die Idee verfolgt, eine weltweite Unterstützung von Servicetechnikern bei der erschwerten Ursachensuche zu ermöglichen. Beispielsweise werden bei einem Auftreten von Betriebs- und Maschinendaten in dem Medizingerät zunächst die bekannten Fakten über das zu analysierende Problem durch das Servicepersonal und/oder einen Bediener des Medizingeräts an die Service- und Entwicklungsingenieure der Entwicklungsabteilung übersendet beziehungsweise diesen mitgeteilt. Die Entwicklungsabteilung kann dann beispielsweise eine mittels einer Konfigurationsvorgabe vorkonfigurierte Vorrichtung an das Servicepersonal übersenden. Alternativ oder zusätzlich ist es möglich, dass die Entwicklungsabteilung die Konfigurationsvorgabe in Form einer Datei an das Servicepersonal übersendet, wobei das Servicepersonal die Konfigurationsvorgabe selbstständig mittels einer Auswertevorrichtung auf die Vorrichtung überträgt. Die Vorrichtung kann, insbesondere mittels der Konfigurationsvorgabe, auf das zu analysierende Problem zugeschnitten sein und/oder zu speichernde Parameter enthalten. Die Konfiguration kann hierbei immer von außen erfolgen und nicht über das Medizingerät selbst, weshalb das Einrichten der Konfigurationsvorgabe auf der Vorrichtung keinen Einfluss auf das Medizingerät hat.

Die Vorrichtung ist bevorzugt kompakt ausgebildet. Beispielsweise ist die Vorrichtung höchstens handtellergroß ausgebildet. Durch die kompakte Ausgestaltung kann die Vorrichtung leicht, beispielsweise per Post, versendet werden und/oder zur Grundausstattung des Servicepersonals zählen. Die einfache Bauweise der Vorrichtung kann eine Verwendung mit verschiedensten Gerätetypen ermöglichen. Neben der Anwendung für die Ursachenanalyse bei Medizingeräten sind auch andere Arten der Datenerfassung, beispielsweise im Rahmen von PMCFU-Studien (PMCFU: Post Market Clinical Follow-Up), möglich.

Gemäß zumindest einer Ausführungsform der Vorrichtung ist die Übertragungseinheit dazu eingerichtet eine Konfigurationsvorgabe von einer Auswertevorrichtung zu empfangen und an die Speichereinheit weiterzuleiten. Beispielsweise wird mittels der Auswertevorrichtung die Konfigurationsvorgabe erstellt und anschließend an die Übertragungseinheit übertragen.

Gemäß zumindest einer Ausführungsform der Vorrichtung ist der zu speichernde Teil der Betriebs- und Maschinendaten mittels der Konfigurationsvorgabe vorgegeben. Die Konfigurationsvorgabe kann insbesondere Informationen über zu erfassende Betriebs- und Maschinendaten des Medizingeräts enthalten. Beispielsweise enthält die Speichereinheit eine Betriebssoftware, die mittels der Konfigurationsvorgabe eingestellt werden kann. Mittels der Betriebssoftware kann die Konfigurationsvorgabe kompiliert werden und zum Beispiel in Steuersignale umgesetzt werden. Beispielsweise können mittels der Auswertevorrichtung Betriebs- und/oder Maschinendaten des Medizingeräts definiert werden, die als zu erfassende Betriebs- und Maschinendaten ausgelesen werden sollen. Dies kann ein selektives Speichern von empfangenen Betriebs- und Maschinendaten des Medizingeräts ermöglichen. Diese Betriebs- und/oder Maschinendaten können in der Konfigurationsvorgabe gespeichert und an die Vorrichtung gesendet werden.

Gemäß zumindest einer Ausführungsform ist das Empfangen der Konfigurationsvorgabe durch die Übertragungseinheit im Betrieb des Medizingeräts blockiert. Hierfür kann die Vorrichtung dazu eingerichtet sein, den Betriebszustand des Medizingeräts übertragen zu bekommen und insbesondere den Betrieb des Medizingeräts zu erkennen. Im Betrieb des Medizingeräts, beispielsweise im Fall einer medizinischen Behandlung, kann dann das Empfangen von Daten mittels der Übertragungseinheit blockiert werden. Das Blockieren kann beispielsweise mit einem Mikrocontroller, der Teil der Übertragungseinheit und/oder der Speichereinheit sein kann, erfolgen. Alternativ oder zusätzlich ist es möglich, dass das Senden mittels der Übertragungseinheit im Betrieb des Medizingeräts blockiert wird. Es kann dann insbesondere jegliche Kommunikation nach außen im Betrieb des Medizingeräts blockiert sein.

Gemäß zumindest einer Ausführungsform der Vorrichtung ist die Vorrichtung dazu eingerichtet, im Betrieb des Medizingeräts mit diesem verbunden und/oder von diesem getrennt zu werden. Insbesondere ist die Vorrichtung dazu eingerichtet, im Betrieb des Medizingeräts mechanisch lösbar und/oder elektrisch leitend mit dem Medizingerät verbunden und/oder von diesem getrennt zu werden. Ferner ist es möglich, dass ein Trennen der Vorrichtung von dem Medizingerät während des Datenauslesevorgangs und/oder während die Vorrichtung mit der Betriebsspannung versorgt wird, erfolgt.

Bevorzugt ist die Vorrichtung dazu eingerichtet, den Betriebszustand des Medizingeräts übertragen zu bekommen und den Betrieb zu erkennen, und daraufhin jegliche leitenden Verbindungen nach außen, bzw. die nicht zu dem Medizingerät führen, elektrisch isolierend zu unterbrechen und/oder jegliche weitere Spannungsquellen abzutrennen.

Gemäß zumindest einer Ausführungsform umfasst die Vorrichtung zumindest einen Steuerausgang, der dazu eingerichtet ist, ein Steuersignal an eine Dateneinheit des Medizingeräts zu senden. Das Steuersignal enthält Informationen und/oder Anweisungen über die von dem Medizingerät bereitzustellenden Betriebs- und Maschinendaten. Das Steuersignal kann beispielsweise durch die Konfigurationsvorgabe vorgegeben sein. Hierbei kann die Speichereinheit dazu eingerichtet sein, das Steuersignal bereitzustellen und mittels des Steuerausgangs an einen Steuereingang des Medizingeräts zu senden, wobei der Steuereingang das Steuersignal an die Dateieinheit übertragen kann. Der Steuereingang und der Steuerausgang können dann dazu eingerichtet sein, insbesondere elektrisch leitend, miteinander verbunden zu werden.

Hierbei und im Folgenden kann es sich bei dem Dateneingang und/oder dem Steuerausgang auch um eine physische Leitung handeln. Der Datenaustausch, das heißt die Übertragung der Betriebs- und Maschinendaten und/oder die Übertragung des Steuersignals, kann auch mittels wenigstens einer, insbesondere zweier, Leitung und/oder mittels eines CAN-Bus erfolgen. Die Kommunikation auf einer Leitung kann bidirektional sein.

Mittels des Steuersignals kann die Vorrichtung zum Beispiel spezielle Betriebs- und Maschinendaten, die zur Ursachenanalyse nützlich sein können, anfordern. Alternativ oder zusätzlich ist es möglich, mittels des Steuersignals Maßnahmen festzulegen, die eine Kommunikationsüberlast bei der Datenabfrage verhindern. Dies wird insbesondere durch den eindeutig identifizierbaren Betriebsmodus des Medizingeräts ermöglicht.

Gemäß zumindest einer Ausführungsform der Vorrichtung weist die Speichereinheit eine Verarbeitungseinheit zum Verarbeiten zumindest eines Teils der Betriebs- und Maschinendaten auf. Das Verarbeiten erfolgt bevorzugt vor dem Speichern des zu speichernden Teils der Betriebs- und Maschinendaten. Bevorzugt handelt es sich bei dem verarbeiteten Teil um den zu speichernden und/oder den gespeicherten Teil der Betriebs- und Maschinendaten. Beispielsweise kann die Verarbeitungseinheit zur Aufbereitung der Betriebs- und Maschinendaten eingerichtet sein. Die Verarbeitung der gespeicherten Betriebs- und Maschinendaten kann ein Komprimieren der Betriebs- und Maschinendaten umfassen. Dies kann eine zeitsparende Datenübertragung ermöglichen.

Ferner ist es möglich, dass zumindest ein Teil der gespeicherten Betriebs- und Maschinendaten bei der Verarbeitung gemittelt und/oder verschlüsselt wird. Die Verarbeitung der Betriebs- und Maschinendaten kann zudem eine Auswertung der Betriebs- und Maschinendaten erfassen. Beispielsweise werden die Betriebs- und Maschinendaten bei einer Auswertung komprimiert und/oder dadurch reduziert, dass nur Betriebs- und Maschinendaten außerhalb eines vorbestimmten Normbereichs gespeichert werden. Alternativ oder zusätzlich kann die Verarbeitungseinheit zumindest einem Teil der Betriebs- und Maschinendaten einen Zeitstempel und/oder ein Datum aufprägen. Hierdurch kann es möglich sein, den Zeitpunkt des Auftretens der Betriebs- und Maschinendaten zu dokumentieren.

Die Übertragungseinheit ist eine drahtlose Übertragungseinheit. Die drahtlose Übertragungseinheit basiert bevorzugt auf einem Low Energy Emission Standard, wie beispielsweise Bluetooth. Hierdurch kann eine elektromagnetische Verträglichkeit und/oder eine Reduktion der elektromagnetischen Belastung ermöglicht werden. Beispielsweise umfasst die Übertragungseinheit einen Bluetooth-Sender. Die Verwendung des Begriffes "Sender" schließt hierbei und im Folgenden nicht die Möglichkeit des Empfangs von Betriebs- und Maschinendaten aus. Hierbei ist es möglich, dass die drahtlose Übertragungseinheit dazu eingerichtet ist, während eines Behandlungsmodus, also insbesondere während einer Behandlung von Patienten, verriegelt beziehungsweise abgeschaltet zu werden. Beispielsweise wird hierfür während der Behandlung ein Signal von dem Medizingerät an die Vorrichtung gesendet. Alternativ oder zusätzlich ist es möglich, dass das Medizingerät ein Datum und/oder ein Zeitstempel überträgt, mittels dem der Betriebszustand beschreiben wird, in dem sich das Medizingerät gerade befindet. Dadurch kann die Vorrichtung erkennen, ob gerade eine Behandlung erfolgt oder nicht. Die Vorrichtung ist dazu konfiguriert, zu überprüfen, ob ein entsprechendes Signal von dem Medizingerät erhalten wurde und im Fall eines entsprechenden Signals die Kommunikation nach außen entweder lediglich auf Senden zu beschränken oder gänzlich zu unterbinden. Dies kann erfolgen, indem die Vorrichtung die Übertragungseinheit teilweise oder ganz deaktiviert wird.

Gemäß zumindest einer Ausführungsform der Vorrichtung ist die drahtlose Übertragungseinheit dazu eingerichtet, Konfigurationsdaten von der Auswertevorrichtung zu empfangen, während die Vorrichtung mit dem Medizingerät verbunden ist. Die drahtlose Übertragungseinheit ist dazu eingerichtet, die gespeicherten Betriebs- und Maschinendaten an die Auswertevorrichtung zu übertragen, während die Vorrichtung mit dem Medizingerät verbunden ist. Beispielsweise können die Übertragungseinheit und/oder die Auswertevorrichtung hierfür eine drahtlose Sendeeinheit, wie beispielsweise einen Bluetooth-Sender, aufweisen. Es kann somit möglich sein, die Konfigurationsvorgabe an die Vorrichtung zu senden oder die gespeicherten Betriebs- und Maschinendaten von dieser auszulesen, ohne die Vorrichtung von dem Medizingerät trennen zu müssen. Die Übertragungseinheit kann dazu eingerichtet sein, das Empfangen der Konfigurationsdaten und/oder das Ausgeben der gespeicherten Betriebs- und Maschinendaten während des Betriebs des Medizingeräts zu ermöglichen. Hierdurch kann ein sicherer und ungestörter Betrieb des Medizingeräts in Verbindung mit einer effizienten Ursachenanalyse gewährleistet werden.

Gemäß zumindest einer Ausführungsform der Vorrichtung ist diese nach Art eines Dongles ausgebildet. Bei einem Dongle kann es sich hierbei und im Folgenden um eine kompakte Hardware-Komponente handeln. Ein Dongle kann kompakt ausgeführt sein. Ein Dongle kann beispielsweise zum Verbinden mit einem System eingerichtet sein, wobei das System durch den Dongle zusätzliche Funktionen erhalten kann. Die Vorrichtung kann dann zum Beispiel höchstens handtellergroß ausgebildet sein. Beispielsweise beträgt eine Ausdehnung der Vorrichtung in jede Raumrichtung maximal 10 cm, bevorzugt maximal 8 cm und besonders bevorzugt maximal 5 cm. Durch die kompakte Ausgestaltung der Vorrichtung weist diese einen geringen Platzbedarf auf. Ferner können sich, insbesondere aufgrund der kompakten Gestaltung, geringere Risiken für eine Kontamination der Vorrichtung, beispielsweise durch Keime und/oder chemische Substanzen, ergeben.

Gemäß zumindest einer Ausführungsform umfasst die Vorrichtung eine erste Verbindungskomponente. Die erste Verbindungskomponente kann eine Kommunikationsschnittstelle und eine Energieversorgungsschnittstelle umfassen. Die Kommunikationsschnittstelle umfasst den Dateneingang und gegebenenfalls und/oder den Steuerausgang, die Energieversorgungsschnittstelle umfasst den Spannungseingang. Bei dem Dateneingang, dem Spannungseingang beziehungsweise dem Steuerausgang kann es sich um Anschlüsse der ersten Verbindungskomponente handeln.

Die erste Verbindungskomponente kann dazu geeignet sein, in eine zweite Verbindungskomponente des Medizingeräts eingesteckt zu werden. Bei der zweiten Verbindungskomponente des Medizingeräts kann es sich um eine Service-Schnittstelle des Medizingeräts handeln. Die erste Verbindungskomponente kann nach Art eines weiblichen Verbinders (Englisch: female connector) ausgebildet sein und die zweite Verbindungskomponente kann nach Art eines, insbesondere mit der ersten Verbindungskomponente zusammenwirkenden, männlichen Verbinders (Englisch: male connector) ausgebildet ist, oder umgekehrt. Beispielsweise ist die erste Verbindungskomponente ein Stecker und die zweite Verbindungskomponente eine Buchse oder umgekehrt.

Beispielsweise handelt es sich bei der ersten Verbindungskomponente und der zweiten Verbindungskomponente um ein D-Sub-Verbindungspaar oder ein USB-Verbindungspaar, wobei ein Verbindungspaar hierbei und im Folgenden die Kombination eines weiblichen und eines männlichen Verbinders einer mechanischen Steckverbindung sein kann. Die erste Verbindungskomponente kann dann beispielsweise ein D-Sub-Stecker oder einen USB-Stecker sein. Insbesondere kann sich die erste Verbindungskomponente für eine CAN-Bus-Verbindung eignen.

Gemäß zumindest einer Ausführungsform ist die erste Verbindungskomponente dazu eingerichtet, die Vorrichtung mit dem Medizingerät mechanisch lösbar und/oder elektrisch leitend zu verbinden. Eine "mechanisch lösbare Verbindung" ist hierbei und im Folgenden eine mechanische Verbindung zwischen zwei Komponenten, die zerstörungsfrei, also ohne Zerstörung der Komponenten, und bevorzugt ohne weitere Werkzeuge, wie beispielsweise Schraubendreher oder Lösungsmittel, gelöst werden kann. Die elektrisch leitende Verbindung kann über den Spannungseingang hergestellt werden. Ferner ist eine elektrisch leitende Verbindung über den Dateneingang, beispielsweise zur Übertragung von elektrischen Datensignalen, möglich.

Gemäß zumindest einer Ausführungsform ist die erste Verbindungskomponente derart ausgebildet, dass eine Erdung der Vorrichtung mit einer Erdung des Medizingeräts verbunden ist. Insbesondere können die Vorrichtung und das Medizingerät auf demselben elektrischen Potential liegen und nach außen abgeschirmt sein.

Die erste Verbindungskomponente kann ein Verbindergehäuse aufweisen, in dem sich die Anschlüsse der ersten Verbindungskomponente befinden. Das Verbindergehäuse kann elektrisch isolierend und/oder elektrisch abschirmend ausgebildet sein. Mittels des Verbindergehäuses kann ein Berührschutz für die Anschlüsse bereitgestellt werden. Das Verbindergehäuse kann mehrteilig oder einteilig ausgebildet sein, wobei die einteilige Ausbildung aufgrund der erleichterten Bedienbarkeit bevorzugt ist. Ein "mehrteiliges Verbindergehäuse" liegt hierbei und im Folgenden vor, wenn das Verbindergehäuse mit mehreren räumlich voneinander getrennten Komponenten gebildet ist oder aus diesen besteht. In diesem Fall kann sich ein Teil der Anschlüsse der ersten Verbindungskomponente, beispielsweise der Dateneingang, in einem ersten Gehäuse befinden, während sich der Rest der Anschlüsse in einem zweiten Gehäuse und/oder gegebenenfalls weiteren Gehäusen der ersten Verbindungskomponente befindet. Bei einem "einteiligen Verbindergehäuse" befinden sich sämtliche Anschlüsse der ersten Verbindungskomponente in einem einzigen Verbindergehäuse. Ein einteiliges Verbindergehäuse kann einstückig ausgebildet sein oder aus mehreren direkt aneinander angrenzenden Teilen bestehen.

Gemäß zumindest einer Ausführungsform der Vorrichtung umfasst diese ein elektrisch isolierend ausgebildetes Gehäuse, das zumindest die Speichereinheit und die Übertragungseinheit enthält. Das Gehäuse kann eine Verkapselung beziehungsweise eine Einkapselung der elektronischen Komponenten der Vorrichtung enthalten oder daraus bestehen. Alternativ oder zusätzlich kann das Gehäuse einen Behälter aus Kunststoff aufweisen oder daraus bestehen. Es ist möglich, dass das Gehäuse direkt an das gegebenenfalls vorhandene Verbindergehäuse der gegebenenfalls vorhandenen ersten Verbindungskomponente angrenzt oder mit diesem einstückig ausgebildet ist. Mittels des Gehäuses können die elektronischen Komponenten der Vorrichtung beispielsweise von äußeren Einflüssen geschützt werden, die potentiell gefährlich für den Betrieb der Vorrichtung und/oder des Medizingeräts sein können. Das Gehäuse kann als Berührschutz der Vorrichtung dienen. Hierdurch kann ein Funkenschlag durch eine Berührung beziehungsweise eine elektrische Kontamination der Vorrichtung und/oder des Medizingeräts vermieden werden.

Es wird ferner ein Medizingerät, bevorzugt zur Durchführung einer Dialysebehandlung, angegeben. Beispielsweise handelt es sich bei dem Medizingerät um ein Dialysegerät zur Überwachung und/oder Durchführung einer Dialysebehandlung. Das Medizingerät ist bevorzugt dazu eingerichtet mit einer hier beschriebenen Vorrichtung zum Übertragen von Betriebs- und Maschinendaten an eine Auswertevorrichtung verbunden zu werden. Das heißt, sämtliche für die Vorrichtung beschriebenen Merkmale sind für das Medizingerät offenbart und umgekehrt.

Gemäß zumindest einer Ausführungsform des Medizingeräts umfasst dieses einen Datenausgang zum Senden von Betriebs- und Maschinendaten des Medizingeräts an eine Vorrichtung, bevorzugt die zuvor beschriebene Vorrichtung. Insbesondere kann der Datenausgang dazu eingerichtet sein, mit dem Dateneingang der zuvor beschriebenen Vorrichtung, bevorzugt mechanisch lösbar und/oder elektrisch leitend, verbunden zu werden.

Gemäß zumindest einer Ausführungsform umfasst das Medizingerät einen Spannungsausgang, der dazu eingerichtet, eine Betriebsspannung zur Versorgung der Vorrichtung auszugeben. Es ist möglich, dass die Betriebsspannung während des gesamten Betriebs des Medizingeräts ausgegeben wird. Alternativ ist es möglich, dass das Medizingerät eine Spannungseinheit zur Überwachung des Spannungsausgangs aufweist. Hierdurch ist es möglich, die Betriebsspannung nur dann auszugeben, wenn die Vorrichtung in das Medizingerät eingesteckt ist und/oder ein Schaltsignal an dem weiteren Anschluss anliegt. Beispielsweise kann das Schaltsignal mit der Vorrichtung an den weiteren Anschluss angelegt werden. Aufgrund der Vermeidung einer konstant an dem Spannungsausgang anliegenden Spannung durch die Verwendung einer Spannungseinheit kann beispielsweise eine Sicherheit im Betrieb des Medizingeräts erhöht werden.

Gemäß zumindest einer Ausführungsform umfasst das Medizingerät eine Dateneinheit, die dazu eingerichtet ist, die Betriebs- und Maschinendaten in Abhängigkeit von einem Steuersignal an dem Datenausgang bereitzustellen. "In Abhängigkeit von einem Steuersignal" bedeutet hierbei und im Folgenden, dass ein Inhalt der an dem Datenausgang durch die Dateneinheit bereitgestellten Vielzahl der Betriebs- und Maschinendaten abhängig von dem Steuersignal variiert wird. Der Inhalt der Vielzahl der Betriebs- und Maschinendaten kann beispielsweise eine Art der Daten, in den Betriebs- und Maschinendaten enthaltende Signale und/oder Parameter, ein Umfang der Betriebs- und Maschinendaten und/oder eine Dauer der Datenausgabe sein. Das Steuersignal kann durch eine in der Vorrichtung, insbesondere der Speichereinheit, gespeicherte Konfigurationsvorgabe, bei der es sich um die im Zusammenhang mit der Vorrichtung beschriebene Konfigurationsvorgabe handeln kann, vorgegeben sein. Beispielsweise umfasst das Medizingerät ferner einen Steuereingang der mit einem Steuerausgang der Vorrichtung verbunden werden kann. Das Steuersignal kann dann von der Vorrichtung über den Steuerausgang bereitgestellt werden und mittels des Steuereingangs des Medizingeräts an die Dateneinheit weitergeleitet werden.

Gemäß zumindest einer Ausführungsform des Medizingeräts umfasst dieses eine zweite Verbindungskomponente. Die zweite Verbindungskomponente weist einen Spannungsausgang auf. Ferner kann die zweite Verbindungskomponente den Datenausgang umfassen. Bevorzugt beinhaltet die zweite Verbindungskomponente eine Vielzahl von Datenausgängen, beispielsweise als Teil eines CAN-Bus-Systems. Die zweite Verbindungskomponente ist dazu eingerichtet, eine erste Verbindungskomponente einer Vorrichtung zum Übertragen von Betriebs- und Maschinendaten des Medizingeräts aufzunehmen beziehungsweise mit der ersten Verbindungskomponente verbunden zu werden. Insbesondere kann das Medizingerät mittels der zweiten Verbindungskomponente und der ersten Verbindungskomponente mit der Vorrichtung mechanisch lösbar und/oder elektrisch leitend verbunden werden. Beispielsweise handelt es sich bei der zweiten Verbindungskomponente um einen CAN-Bus-Verbinder, der zumindest einen CAN-Bus, insbesondere zwei CAN-Busse, enthalten kann.

Es wird ferner ein Verfahren zum Übertragen von Betriebs- und Maschinendaten eines Medizingeräts, bevorzugt eines Dialysegeräts, an eine Auswertevorrichtung angegeben. Bevorzugt wird das Verfahren zum Übertragen von Betriebs- und Maschinendaten eines hier beschriebenen Medizingeräts verwendet. Ferner wird das Verfahren bevorzugt mit einer hier beschriebenen Vorrichtung durchgeführt. Das heißt, sämtliche für die Vorrichtung und/oder das Medizingerät offenbarten Merkmale sind für das Verfahren offenbart und umgekehrt.

Gemäß zumindest einer Ausführungsform des Verfahrens wird eine Vorrichtung bereitgestellt. Die Vorrichtung umfasst einen Dateneingang, eine programmierbare Speichereinheit, einen Spannungseingang und eine Übertragungseinheit. Bei der Vorrichtung handelt es sich bevorzugt um die zuvor beschriebene Vorrichtung.

Gemäß zumindest einer Ausführungsform des Verfahrens wird die Vorrichtung, bevorzugt mechanisch lösbar und/oder elektrisch leitend, mit dem Medizingerät verbunden. Das Verbinden erfolgt derart, dass die Vorrichtung über den Spannungseingang mit einer von dem Medizingerät bereitgestellten Betriebsspannung versorgt wird. Das Verbinden kann ferner mittels der gegebenenfalls vorhandenen ersten Verbindungskomponente der Vorrichtung, beispielsweise durch Einstecken der ersten Verbindungskomponente in eine zweite Verbindungskomponente des Medizingeräts, erfolgen. Das Verbinden der Vorrichtung mit dem Medizingerät kann zudem derart erfolgen, dass der Dateneingang mit einem Datenausgang des Medizingeräts verbunden ist. Alternativ oder zusätzlich kann das Verbinden derart erfolgen, dass ein Spannungseingang der Vorrichtung mit einem Spannungsausgang des Medizingeräts verbunden ist.

Das Verfahren beinhaltet ein Empfangen von Betriebs- und Maschinendaten des Medizingeräts mit dem Dateneingang. Die Betriebs- und Maschinendaten können beispielsweise an dem Datenausgang des Medizingeräts bereitgestellt werden. Ein Teil der Betriebs- und Maschinendaten wird mittels der Speichereinheit gespeichert. Beispielsweise können die Betriebs- und Maschinendaten vor dem Speichern mit einer Verarbeitungseinheit der Vorrichtung verarbeitet werden.

Die gespeicherten Betriebs- und Maschinendaten werden an eine Auswertevorrichtung übertragen. Das Übertragen erfolgt mittels der Übertragungseinheit. Das Übertragen wird durchgeführt, während die Vorrichtung mit dem Medizingerät verbunden ist. In einer nicht erfindungsgemäßen Alternative ist es möglich, dass die Vorrichtung vor dem Übertragen von dem Medizingerät getrennt wird und anschließend, insbesondere mechanisch lösbar und/oder elektrisch leitend, mit der Auswertevorrichtung verbunden wird.

Gemäß zumindest einer Ausführungsform des Verfahrens wird die Vorrichtung mittels des Spannungseingangs mit einer von dem Medizingerät über den Spannungsausgang bereitgestellten Betriebsspannung versorgt. Bevorzugt wird die Vorrichtung ausschließlich mittels der an dem Spannungseingang anliegenden Betriebsspannung versorgt, wenn sie mit dem Medizingerät verbunden ist.

Gemäß zumindest einer Ausführungsform umfasst das Verfahren ferner die Schritte eines Verbindens der Vorrichtung mit einer Auswertevorrichtung, ein Übertragen einer Konfigurationsvorgabe von der Auswertevorrichtung an die Speichereinheit. Die Schritte werden bevorzugt mittels der Übertragungseinheit durchgeführt. Hierfür wird eine drahtlose Verbindung zwischen der Übertragungseinheit und der Auswertevorrichtung hergestellt. Die Übertragungseinheit ist eine drahtlose Übertragungseinheit und die Auswertevorrichtung kann eine drahtlose Sendeeinheit aufweisen. Mittels der Übertragungseinheit kann die Konfigurationsvorgabe dann auf die Vorrichtung übertragen werden und die Konfigurationsvorgabe an die Speichereinheit weitergeleitet werden. Bevorzugt wird die Vorrichtung nach dem Übertragen der Konfigurationsvorgabe von der Auswertevorrichtung getrennt.

Gemäß zumindest einer Ausführungsform des Verfahrens wird das Übertragen der Konfigurationsvorgabe, und insbesondere das Verbinden der Vorrichtung mit der Auswertevorrichtung, durchgeführt, während die Vorrichtung mit dem Medizingerät verbunden ist. Das Übertragen der gespeicherten Betriebs- und Maschinendaten an die Auswertevorrichtung wird durchgeführt, während die Vorrichtung mit dem Medizingerät verbunden ist. Mit anderen Worten, erfolgt das Übertragen der gespeicherten Betriebs- und Maschinendaten und gegebenenfalls das Übertragen der Konfigurationsvorgabe nach dem Verbinden der Vorrichtung mit dem Medizingerät und vor einem Trennen der Vorrichtung von dem Medizingerät. Das Übertragen der Konfigurationsvorgabe und/oder der gespeicherten Betriebs- und Maschinendaten kann insbesondere während des Betriebs des Medizingeräts erfolgen.

Die Übertragungseinheit ist eine drahtlose Übertragungseinheit, wobei die gespeicherten Betriebs- und Maschinendaten an die Auswertevorrichtung mittels der drahtlosen Übertragungseinheit gesendet werden, während die Vorrichtung mit dem Medizingerät verbunden ist. Ferner kann die Konfigurationsvorgabe mittels der drahtlosen Übertragungseinheit an die Vorrichtung übertragen werden, während die Vorrichtung mit dem Medizingerät verbunden ist. Hierzu kann die Auswertevorrichtung eine drahtlose Sendeeinheit aufweisen, die die Konfigurationsdaten an die Übertragungseinheit sendet.

Gemäß zumindest einer Ausführungsform des Verfahrens erfolgt das Verbinden der Vorrichtung mit dem Medizingerät während des Betriebs des Medizingeräts. Alternativ oder zusätzlich erfolgt ein gegebenenfalls in einem weiteren Schritt durchgeführtes Trennen der Vorrichtung von dem Medizingerät während des Betriebs des Medizingeräts. Das Verbinden und/oder Trennen der Vorrichtung mit beziehungsweise von dem Medizingerät hat somit bevorzugt keinen Einfluss auf den Betrieb, und insbesondere die Betriebssicherheit, des Medizingeräts. Die Vorrichtung kann also beispielsweise während der Durchführung einer medizinischen Behandlung, insbesondere einer Dialysebehandlung, in das Medizingerät eingesteckt werden oder von diesem getrennt werden. Hierdurch wird eine Ursachenanalyse und -behebung auch ohne ein langwieriges und mitunter nicht ohne weiteres mögliches Beenden der Behandlung ermöglicht. Insbesondere können während der Behandlung auftretende Betriebs- und Maschinendaten instantan untersucht werden.

Gemäß zumindest einer Ausführungsform des Verfahrens umfasst dieses ferner ein Bereitstellen der Auswertevorrichtung, die eine Eingabeschnittstelle aufweist. Die Eingabeschnittstelle kann beispielsweise eine Tastatur und/oder ein Touchscreen in Verbindung mit einer, insbesondere grafischen, Applikationssoftware der Auswertevorrichtung, wie beispielsweise eine App, sein. Ferner umfasst das Verfahren ein Eingeben der Konfigurationsdaten in die Auswertevorrichtung mittels der Eingabeschnittstelle. Beispielsweise sind alle möglichen Konfigurationsvorgaben in einer Art grafischen Checkliste auf einem Bildschirm der Auswertevorrichtung dargestellt und können von einem Benutzer, wie zum Beispiel dem Servicepersonal oder der Entwicklungsabteilung, bereitgestellt werden.

Gemäß zumindest einer Ausführungsform des Verfahrens umfasst dieses ferner ein weiteres Verarbeiten der gespeicherten Betriebs- und Maschinendaten mittels der Auswertevorrichtung. Die Auswertevorrichtung kann beispielsweise identisch mit der Auswertevorrichtung sein. Beispielsweise werden die gespeicherten Betriebs- und Maschinendaten mittels der Auswertevorrichtung grafisch dargestellt, gespeichert, komprimiert, verwaltet und/oder anderweitig weiterverarbeitet. Die Auswertevorrichtung kann ferner einen Internetzugang und/oder einen Faxzugang aufweisen. Beispielsweise ist es hierdurch möglich, die gespeicherten Betriebs- und Maschinendaten per E-Mail und/oder per Fax zu versenden und/oder mittels der Auswertevorrichtung auf eine Informationsdatenbank des Herstellers des Medizingeräts zuzugreifen. Beispielsweise kann die Auswertevorrichtung zum Weiterverarbeiten der gespeicherten Betriebs- und Maschinendaten eine Applikationssoftware aufweisen. Es ist insbesondere möglich, dass das Übertragen der gespeicherten Betriebs- und Maschinendaten simultan zum Speichern von, insbesondere weiteren, Betriebs- und Maschinendaten erfolgt. Hierdurch kann zum Beispiel bereits während des Auslesens der Betriebs- und Maschinendaten überprüft werden, ob die Konfigurationsvorgabe zur Ursachenanalyse geeignet ist und die Konfigurationsvorgabe gegebenenfalls angepasst werden.

### Kurze Beschreibung der Figuren

Im Folgenden wird die hier beschriebene Vorrichtung, das hier beschriebene Medizingerät und das hier beschriebene Verfahren anhand von Ausführungsbeispielen und den dazugehörigen Figuren näher erläutert.
- Die Figuren 1 und 2: zeigen schematisch Ausführungsbeispiele einer hier beschriebenen Vorrichtung anhand schematischer Darstellungen.
- Die Figuren 3 und 4: zeigen schematisch Ausführungsbeispiele einer hier beschriebenen Vorrichtung und eines hier beschriebenen Medizingeräts anhand schematischer Darstellungen.
- Die Figur 5: zeigt ein Ausführungsbeispiel eines hier beschriebenen Verfahrens anhand einer schematischen Darstellung.

### Ausführliche Beschreibung der Figuren

Gleiche, gleichartige oder gleich wirkende Elemente sind in den Figuren mit denselben Bezugszeichen versehen. Die Figuren und die Größenverhältnisse der in den Figuren dargestellten Elemente untereinander sind nicht als maßstäblich zu betrachten. Vielmehr können einzelne Elemente zur besseren Darstellbarkeit und/oder zum besseren Verständnis übertrieben groß dargestellt sein.

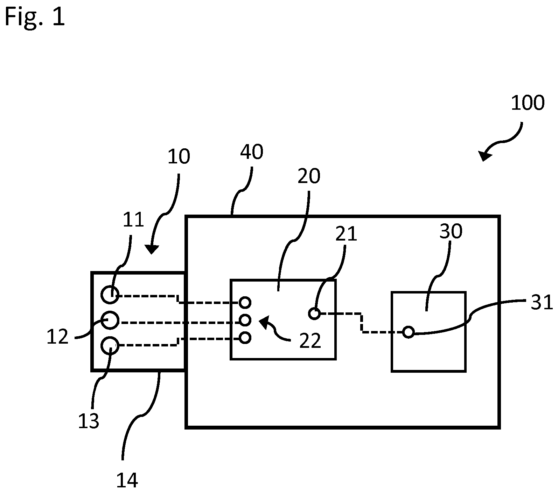

Anhand der schematischen Darstellung der Figur 1 ist ein Ausführungsbeispiel einer hier beschriebenen Vorrichtung 100 näher erläutert. Die Vorrichtung 100 ist in dem Ausführungsbeispiel der Figur 1 nach Art eines Dongles ausgebildet, welches an einem entsprechenden Datenausgang zur Übertragung von Betriebs- und Maschinendaten eines Medizingeräts angeschlossen werden kann.

Die Vorrichtung 100 dient zum Speichern von Betriebs- und Maschinendaten eines Medizingeräts 500, das beispielsweise ein Dialysegerät sein kann. Die Vorrichtung kann ferner dazu konfiguriert sein, Betriebs- und Maschinendaten an eine Auswertevorrichtung zu übertragen. Die Vorrichtung umfasst einen Dateneingang 12 zum Empfangen von Betriebs- und Maschinendaten des Medizingeräts 500, eine programmierbare Speichereinheit 20, die dazu eingerichtet ist, zumindest einen Teil der empfangenen Betriebs- und Maschinendaten zu speichern, und eine Übertragungseinheit 30, die dazu eingerichtet ist, die gespeicherten Betriebs- und Maschinendaten an die Auswertevorrichtung zu übertragen. Ferner ist ein Spannungseingang 11, der dazu eingerichtet ist, die Vorrichtung 100 mit einer von dem Medizingerät 500 bereitgestellten Betriebsspannung zu versorgen, vorhanden.

Die programmierbare Speichereinheit 20 umfasst insbesondere eine Speichervorrichtung, beispielsweise einen nichtflüchtigen Speicher und einen Prozessor bzw. Mikrochip. Der Prozessor bzw. Mikrochip ist dazu eingerichtet, den Speichervorgang zu unterstützen und/oder Daten zu verarbeiten und/oder die Kommunikation zu übernehmen etc. Der Prozessor bzw. Mikrochip kann durch Konfigurationsvorgaben programmiert werden und dient dann unter anderem auch als Verarbeitungseinheit zum Verarbeiten von Betriebs- und Maschinendaten.

Die Vorrichtung 100 umfasst zudem eine erste Verbindungskomponente 10 mit dem Spannungseingang 11 sowie Signalleitungen 12 ,13. Die Signalleitungen 12, 13 können ein Dateneingang 12 und einem Steuerausgang 13 sein. Es ist aber auch möglich, dass es sich bei den Signalleitungen 12, 13 um eine, insbesondere einzige, bidirektionale Schnittstelle handelt, wie beispielweise bei einem CAN-Bus. Hierbei ist rein exemplarisch jeweils nur ein Anschluss dargestellt. Bei der ersten Verbindungskomponente 10 handelt es sich in dem dargestellten Ausführungsbeispiel um einen Stecker, der je nach Ausprägung der dazu korrespondierenden Buchse des Medizingeräts ausgebildet ist.

Die erste Verbindungskomponente 10 kann insbesondere auch eine Vielzahl von Dateneingängen 12 und/oder Steuerausgängen 13 aufweisen, welche mit den am jeweiligen Medizingerät vorliegenden Datenausgängen und Steuereingängen korrespondieren. Beispielsweise kann die Verbindungskomponente 10 separate Signalleitungen 12, 13 für eine Betriebssteuerungskommunikation und für eine Schutzsystemkommunikation des Medizingerätes aufweisen. Durch eine solche Ausgestaltung kann die Vorrichtung 100 an die interne Kommunikationsstruktur des Medizingerätes angepasst sein. Beispielsweise weist das Medizingerät zwei separate CAN-Busse auf - einen für die Betriebssteuerungskommunikation des Medizingerätes und einen weiteren für die Schutzsystemkommunikation des Medizingerätes.

Die erste Verbindungskomponente 10 kann auch mit Dateneingängen 12 und/oder Steuerausgängen 13 für unterschiedliche Medizingeräte ausgeführt sein, so dass die Vorrichtung 100 zum Übertragen von Betriebs- und Maschinendaten unterschiedlicher Medizingerätetypen ausgebildet sein kann.

Ferner ist auch der Spannungseingang 11 schematisch als einzelner Anschluss dargestellt, beinhaltet jedoch wenigstens zwei Pole und gegebenenfalls eine Erdung, um die Spannungsversorgung der Vorrichtung 100 bei deren Anschluss an das Medizingerät sicher zu stellen. Der Spannungseingang 11 kann ebenfalls so ausgebildet sein, dass er zu mehreren Medizingeräten kompatibel ist.

Die erste Verbindungskomponente 10 beinhaltet ferner ein Verbindergehäuse 14, das die übrigen Komponenten der ersten Verbindungskomponente 10 umgibt und miteinander verbindet. In dem Ausführungsbeispiel der Figur 1 ist das Verbindergehäuse 14 einteilig ausgebildet. Sämtliche Anschlüsse der ersten Verbindungskomponente 10 befinden sich also in einem einzigen Verbindergehäuse 14. Das Verbindergehäuse 14 und die darin angeordneten Anschlüsse bilden gemeinsam eine Steckverbindung aus, die mit der von dem Medizingerät bereitgestellten Buchse korrespondiert.

Die Vorrichtung 100 umfasst ferner ein Gehäuse 40, an dem sich das Verbindergehäuse 14 und in dem sich die Speichereinheit 20 und die Übertragungseinheit 30 befinden.

Die Speichereinheit 20 ist mit dem Spannungseingang 11, dem Dateneingang 12 und dem Steuerausgang 13 der ersten Verbindungskomponente 10 verbunden. In der Figur 1 nicht gezeigt ist eine mögliche elektrische Verbindung zwischen dem Spannungseingang 11 und der Übertragungseinheit 30, über welche auch die Übertragungseinheit 30 mit elektrischer Energie versorgt werden kann. Damit kann die Speichereinheit 20 Betriebs- und Maschinendaten, die über den Dateineingang 12 vom Medizingerät geliefert werden, speichern. Die Betriebs- und Maschinendaten können dabei am Dateneingang 12 von dem Medizingerät über einen vorbestimmten Zeitraum hinweg empfangen werden und in der Speichereinheit 20 gespeichert werden. Entsprechend werden die Betriebs- und Maschinendaten in der Speichereinheit 20 kumuliert.

In einer bevorzugten Ausführungsform werden die Betriebs- und Maschinendaten, die von dem Medizingerät empfangen werden, jeweils mit einem Zeitstempel versehen, wenn sie in der Speichereinheit 20 gespeichert werden.

Die Speichereinheit 20 ist über einen ersten Übertragungsanschluss 21 mit einem zweiten Übertragungsanschluss 31 der Übertragungseinheit 30 verbunden. Entsprechend kann die Übertragungseinheit 30 mit der Speichereinheit 20 kommunizieren und von der Speichereinheit 20 die in der Speichereinheit gespeicherten Betriebs- und Maschinendaten übernehmen und dann an eine hier nicht gezeigte Auswertevorrichtung übertragen.

Wenn in der Speichereinheit 20 die Betriebs- und Maschinendaten über einen vorbestimmten Zeitraum hinweg gespeichert werden, kann mit der Übertragungseinheit 30 eine zeitversetzte Übertragung der Betriebs- und Maschinendaten an die Auswertevorrichtung vorgenommen werden. Es werden dann insbesondere die über einen vorbestimmten Zeitraum hinweg gespeicherten Betriebs- und Maschinendaten auf einmal an die Auswertevorrichtung übertragen - beispielsweise immer dann, wenn ein Servicetechniker anwesend ist und die Übertragungseinheit 30 mit einer Auswertevorrichtung des Servicetechnikers in Kommunikation steht. Die Übertragung kann zu bestimmten Zeitpunkten erfolgen, beispielsweise wenn das Medizingerät signalisiert, dass eine Behandlung beendet wurde.

Die Übertragungseinheit 30 ist als drahtlose Übertragungseinheit 30 ausgebildet, mittels welcher die in der Speichereinrichtung 20 gespeicherten Betriebs- und Maschinendaten drahtlos an die Auswertevorrichtung übertragen werden können. Als Übertragungsprotokolle bieten sich dabei solche an, die eine niedrige elektromagnetische Emission aufweisen, beispielsweise kurzreichweitige Übertragungsprotokolle wie Bluetooth.

Die Übertragungseinheit 30 kann gemäß einer nicht erfindungsgemäßen Alternative aber auch in Form einer Steckverbindung ausgebildet sein, wobei auch die durch die erste Verbindungskomponente 10 ausgebildete Steckverbindung zur Übertragung der Betriebs- und Maschinendaten mittels der Übertragungsvorrichtung 30 dienen kann.

Die Vorrichtung 100 kann weitere Komponenten, insbesondere weitere Anschlüsse, aufweisen.

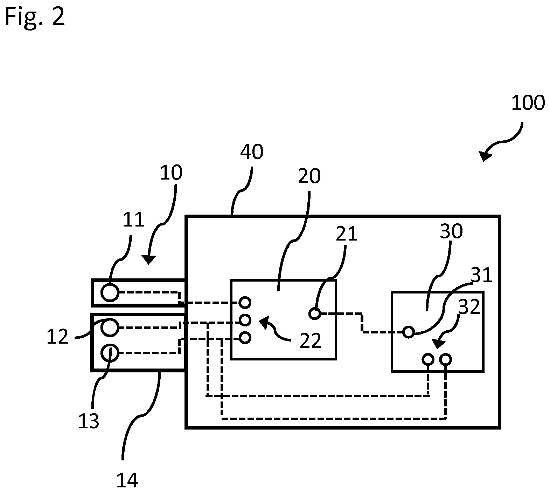

Anhand der schematischen Darstellung der Figur 2 ist ein weiteres Ausführungsbeispiel einer hier beschriebenen Vorrichtung 100 näher erläutert. Das Ausführungsbeispiel der Figur 2 unterscheidet sich von dem Ausführungsbeispiel der Figur 1 in der mechanischen Ausbildung der ersten Verbindungskomponente 10 wie folgt.

Zunächst ist das Verbindergehäuse 14 der Vorrichtung 100 des Ausführungsbeispiels der Figur 2 mehrteilig ausgebildet. Insbesondere befindet sich der Spannungseingang 11 in einem ersten Teil des Verbindergehäuses und der Dateneingang 12 und der Signalausgang 13 in einem zweiten Teil des Verbindergehäuses. Die beiden Teile des Verbindergehäuses sind räumlich voneinander getrennt und/oder elektrisch voneinander isoliert ausgebildet. Alternativ oder zusätzlich zu der Darstellung der Figur 2 ist es möglich, dass sich ein Teil des Verbindergehäuses an einer einem anderen Teil des Verbindergehäuses abgewandten Seite des Gehäuses 40 der Vorrichtung befindet.

Bei dem Ausführungsbeispiel der Figur 2 weist die Übertragungseinheit 30 zudem Anschlüsse 32 auf, welche mit dem Dateneingang 12 und dem Steuerausgang 13 verbunden sind. Beispielsweise wird hierdurch eine nicht erfindungsgemäße drahtgebundene Verbindung der Vorrichtung 100 mit der Auswertevorrichtung mittels der ersten Verbindungskomponente 10 ermöglicht. Alternativ kann die Vorrichtung 100 eine weitere Verbindungskomponente, beispielsweise einen weiteren Stecker, aufweisen, die zur Verbindung der Vorrichtung 100 mit der Auswertevorrichtung dienen kann.

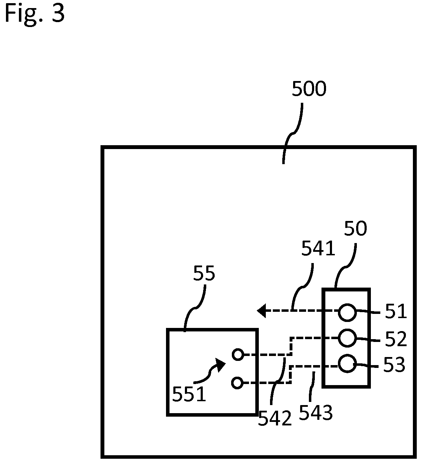

Anhand der schematischen Darstellung der Figur 3 ist ein Ausführungsbeispiel eines Medizingeräts 500 näher erläutert. Bevorzugt ist das Medizingerät 500 ein Dialysegerät zur Durchführung einer Dialysebehandlung, beispielsweise zur Durchführung einer Hämodialyse, einer Hämofiltration, einer Hämodiafiltration, einer Peritonealdialyse, einer Akutdialyse, einer Leberersatztherapie, einer Apharese und/oder einer anderen Dialysebehandlung.

Das Medizingerät 500 umfasst eine zweite Verbindungskomponente 50 mit einem Spannungsausgang 51, einem Datenausgang 52 und einem Steuereingang 53. Die zweite Verbindungskomponente 50 kann weitere (in den Figuren nicht dargestellte) Datenausgänge und Steuereingänge umfassen. Ferner beinhaltet das Medizingerät 500 eine erste Verbindung 541, eine zweite Verbindung 542, eine dritte Verbindung 543, eine Dateneinheit 55 und Datenanschlüsse 551.

Die zweite Verbindungskomponente 50 ist zur Aufnahme einer ersten Verbindungskomponente 10 einer Vorrichtung 100, insbesondere der Vorrichtung 100 des Ausführungsbeispiels der Figur 1, ausgebildet. Insbesondere sind der Spannungsausgang 51, der Datenausgang 52 beziehungsweise der Steuereingang 53 zur Verbindung mit einem Spannungseingang 11, einem Dateneingang 12 beziehungsweise einem Steuerausgang 13 der Vorrichtung 100 ausgebildet.

Mittels des Medizingeräts 500 kann über die erste Verbindung 541 an dem Spannungsausgang 51 eine Betriebsspannung angelegt werden, die zur Energieversorgung einer Vorrichtung 100 geeignet ist. Über die zweite Verbindung 542 beziehungsweise die dritte Verbindung 543 sind der Datenausgang 52 beziehungsweise der Steuereingang 53 mit der Dateneinheit 55 verbunden.

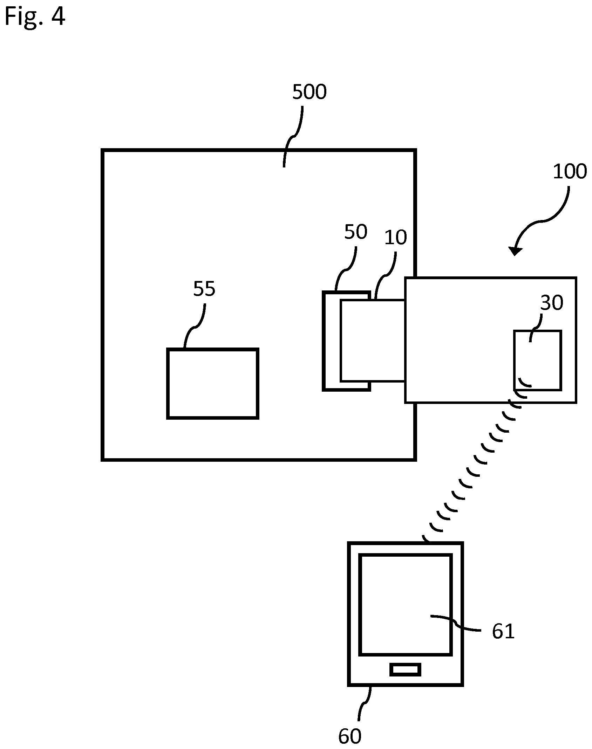

Anhand der schematischen Darstellung der Figur 4 ist ein Ausführungsbeispiel einer hier beschriebenen Vorrichtung 100 und eines hier beschriebenen Medizingeräts 500 näher erläutert. Dargestellt ist eine Vorrichtung 100 gemäß dem Ausführungsbeispiel der Figur 1, die mittels der ersten Verbindungskomponente 10 in die zweite Verbindungskomponente 50 eines Medizingeräts 500 gemäß dem Ausführungsbeispiel der Figur 3 eingesteckt ist. Zur Übersichtlichkeit sind die Anschlüsse der Vorrichtung 100 und insbesondere der ersten Verbindungskomponente 10 beziehungsweise des Medizingeräts 500 und insbesondere der zweiten Verbindungskomponente 50 in der Figur 4 nicht dargestellt.

Ferner ist eine Auswertevorrichtung 60 vorhanden, die eine drahtlose Sendeeinheit und eine Eingabeschnittstelle 61 beinhaltet. Die Auswertevorrichtung 60 kann beispielsweise in Form eines mobilen Endgeräts wie beispielsweise eines Smartphones oder eines Tablets vorgesehen sein. Die Auswertevorrichtung 60 steht damit nicht im elektrischen Kontakt mit dem Medizingerät 500, sondern erhält die in der Speichereinrichtung 20 gespeicherten Betriebs- und Maschinendaten über die Übertragungseinheit 30 drahtlos.

Ist in einer nicht erfindungsgemäße Alternative keine drahtlose Schnittstelle vorhanden und findet die Übertragung über den Stecker der ersten Verbindungskomponente 10 statt, indem die Vorrichtung 100 in die Auswertevorrichtung 60 eingesteckt wird, so steht die Auswertevorrichtung 60 ebenfalls nicht in elektrischem Kontakt mit dem Medizingerät 500, da die Vorrichtung 100 nur entweder in dem Medizingerät 500 oder in der Auswertevorrichtung 60 eingesteckt werden kann.

Entsprechend findet ein zeitversetztes beziehungsweise asynchrones Übertragen von Betriebs- und Maschinendaten von dem Medizingerät 500 an die Auswertevorrichtung 60 statt, ohne dass ein elektrischer Kontakt zwischen der Auswertevorrichtung 60 und dem Medizingerät 500 besteht.

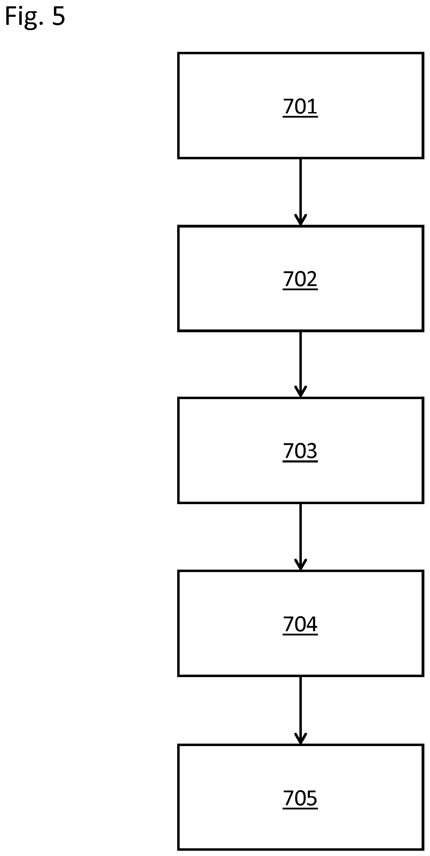

Anhand der schematischen Darstellung der Figur 5 ist ein Ausführungsbeispiel eines hier beschriebenen Verfahrens näher erläutert. Das Übertragen der Betriebs- und Maschinendaten des Medizingeräts 500 an die Auswertevorrichtung 60 kann beispielsweise wie folgt erfolgen. Mittels der Auswertevorrichtung 60 wird in einem ersten Verfahrensschritt 701 eine Konfigurationsvorgabe an die drahtlose Übertragungseinheit 30 der Vorrichtung 100 gesendet und von dieser an die Speichereinheit 20 weitergeleitet. In der Speichereinheit 20 wird die Konfigurationsvorgabe in einem zweiten Verfahrensschritt 702 kompiliert und ein Steuersignal erzeugt, das über den Steuerausgang 13 der Vorrichtung 100 an den Steuereingang 53 des Medizingeräts 500 gesendet wird und über die erste Verbindung 541 an die Dateneinheit 55 weitergeleitet wird. Entsprechend des Steuersignals stellt die Dateneinheit 55 Betriebs- und Maschinendaten bereit, die sie über die zweite Verbindung 542 an den Datenausgang 52 ausgibt, wo die Betriebs- und Maschinendaten mittels des Dateneingangs 12 der Vorrichtung 100 an die Speichereinheit 20 weitergeleitet werden (dritter Verfahrensschritt 703). Gegebenenfalls extrahiert die Speichereinheit 20 nun einen Teil der Betriebs- und Maschinendaten aus den empfangenen Betriebs- und Maschinendaten und speichert diesen Teil. Das Extrahieren beziehungsweise Herausfiltern der zu speichernden Betriebs- und Maschinendaten aus der Vielzahl der empfangenen von Betriebs- und Maschinendaten erfolgt anhand der Konfigurationsvorgabe. Es ist jedoch auch möglich, dass die Speichereinheit 100 die Vielzahl von Betriebs- und Maschinendaten vollständig als gespeicherte Betriebs- und Maschinendaten speichert.

Alternativ ist es möglich, dass kein Steuersignal an die Dateneinheit 55 gesendet wird beziehungsweise keine Dateneinheit 55 vorhanden ist. In diesem Fall stellt das Medizingerät 500 stets dieselben Betriebs- und Maschinendaten, unabhängig von der Konfigurationsvorgabe, bereit. Die Speichereinheit 20 extrahiert dann den zu speichernden Teil der Betriebs- und Maschinendaten anhand der Konfigurationsvorgabe.

In einem zusätzlichen vierten Verfahrensschritt 704, der bevorzugt vor dem Speichern durchgeführt wird, können die gespeicherten Betriebs- und Maschinendaten gegebenenfalls verarbeitet oder anderweitig aufbereitet werden, wie beispielsweise durch eine Verschlüsselung oder eine Mittelung der Betriebs- und Maschinendaten. Die so bearbeiteten und, bevorzugt anschließend, gespeicherten Betriebs- und Maschinendaten werden dann in einem fünften Verfahrensschritt 705 mittels der Übertragungseinheit 30 an die Auswertevorrichtung übertragen. Das Übertragen der gespeicherten Betriebs- und Maschinendaten kann während des Datenauslesevorgangs erfolgen. Alternativ kann das Übertragen zeitverzögert, also nachdem das Speichern vollständig abgeschlossen ist, erfolgen.

In einer weiteren bevorzugten Ausführung werden alle während des Betriebs des Medizingeräts 500 im Medizingerät 500 erzeugten Betriebs- und Maschinendaten am Datenausgang 52 des Medizingeräts 500 von der Vorrichtung 100 ausgelesen und an deren Dateneingang 12 empfangen. Die Übermittlung von Steuerdaten von der Vorrichtung 100 an das Medizingerät 500 ist damit nicht notwendig. Entsprechend "horcht" die Vorrichtung 100 nur die im Medizingerät 500 erzeugten Betriebs- und Maschinendaten ab, beispielsweise durch das Abhören eines entsprechenden Busses. Auf die Ausbildung des Steuerausgangs 13 der ersten Verbindungskomponente 10 der Vorrichtung 100 und damit auch auf die Ausbildung des Steuereingangs 53 im Medizingerät 500 kann entsprechend verzichtet werden.

### Bezugszeichenliste

- 100: Vorrichtung
- 10: erste Verbindungskomponente
- 11: Spannungseingang
- 12: Dateneingang
- 13: Steuerausgang
- 14: Verbindergehäuse
- 20: Speichereinheit
- 21: erster Übertragungsanschluss
- 22: Anschlüsse
- 30: Übertragungseinheit
- 31: zweiter Übertragungsanschluss
- 32: Anschlüsse der Übertragungseinheit
- 40: Gehäuse
- 500: Medizingerät
- 50: zweite Verbindungskomponente
- 51: Spannungsausgang
- 52: Datenausgang
- 53: Steuereingang
- 541: erste Verbindung
- 542: zweite Verbindung
- 543: dritte Verbindung
- 55: Dateneinheit
- 551: Datenanschlüsse
- 60: Auswertevorrichtung
- 61: Eingabeschnittstelle
- 701: erster Verfahrensschritt
- 702: zweiter Verfahrensschritt
- 703: dritter Verfahrensschritt
- 704: vierter Verfahrensschritt
- 705: fünfter Verfahrensschritt

## Patentansprüche

1. Vorrichtung (100) zum Übertragen von Betriebs- und Maschinendaten eines Medizingeräts (500), bevorzugt eines Dialysegeräts, an eine Auswertevorrichtung (60), wobei die Vorrichtung (100)
- einen Dateneingang (12) zum Empfangen von Betriebs- und Maschinendaten des Medizingeräts (500),
- eine programmierbare Speichereinheit (20), die dazu eingerichtet ist, zumindest einen Teil der empfangenen Betriebs- und Maschinendaten zu speichern,
- einen Spannungseingang (11), der dazu eingerichtet ist, die Vorrichtung (100) mit einer von dem Medizingerät (500) bereitgestellten Betriebsspannung zu versorgen, und
- eine Übertragungseinheit (30), die dazu eingerichtet ist, die gespeicherten Betriebs- und Maschinendaten an die Auswertevorrichtung (60) zu übertragen, umfasst,
wobei die Übertragungseinheit (30) dazu eingerichtet ist, die in der Speichereinheit (20) gespeicherten Betriebs- und Maschinendaten drahtlos an die Auswertevorrichtung (60) zu übertragen während die Vorrichtung (100) mit dem Medizingerät (500) verbunden ist, und wobei die Vorrichtung (100) dazu eingerichtet ist, die Kommunikation nach außen auf Senden zu beschränken oder zu unterbinden, wenn ein dem Betriebszustand des Medizingeräts (500) entsprechendes Signal von dem Medizingerät übertragen wird.

2. Vorrichtung (100) nach Anspruch 1, wobei das Signal ein(en) den Betriebszustand beschreibendes Datum und/oder Zeitstempel umfasst und/oder wobei das Signal ein Vorliegen eines aktuellen Behandlungsmodus beschreibt.

3. Vorrichtung (100) nach Anspruch 1 oder 2, wobei
- die Übertragungseinheit (30) dazu eingerichtet ist, eine Konfigurationsvorgabe von der Auswertevorrichtung (60) zu empfangen und an die programmierbare Speichereinheit (20) weiterzuleiten und
- die programmierbare Speichereinheit (20) durch die Konfigurationsvorgabe dazu eingerichtet ist, Betriebs- und Maschinendaten gemäß der Konfigurationsvorgabe zu speichern.

4. Vorrichtung (100) nach dem vorherigen Anspruch, wobei das Empfangen der Konfigurationsvorgabe durch die Übertragungseinheit (30) im Betrieb des Medizingeräts (500) blockiert ist.

5. Vorrichtung (100) nach einem der vorhergehenden Ansprüche, wobei die Vorrichtung (100) dazu eingerichtet ist, im Betrieb des Medizingeräts (500) mit dem Medizingerät (500) verbunden und/oder von diesem getrennt zu werden.

6. Vorrichtung (100) nach einem der vorhergehenden Ansprüche, wobei die Speichereinheit (20) eine Verarbeitungseinheit zum Verarbeiten zumindest eines Teils der Betriebs- und Maschinendaten aufweist.

7. Vorrichtung (100) nach einem der vorhergehenden Ansprüche, wobei die Vorrichtung (100) nach Art eines Dongles ausgebildet ist.

8. Vorrichtung (100) nach einem der vorhergehenden Ansprüche, umfassend ein elektrisch isolierend ausgebildetes Gehäuse (40), das die Speichereinheit (20) und die Übertragungseinheit (30) enthält.

9. System umfassend eine Vorrichtung (100) gemäß einem der vorstehenden Ansprüche und ein Medizingerät (500), bevorzugt zur Durchführung einer Dialysebehandlung, wobei die Vorrichtung (100) eine erste Verbindungskomponente (10) aufweist und das Medizingerät (500) eine zweite Verbindungskomponente (50) aufweist, und die Vorrichtung (100) mittels der ersten Verbindungskomponente (10) in die zweite Verbindungskomponente (50) des Medizingeräts (500) eingesteckt und mit dieser verbunden ist, und wobei das Medizingerät (500) einen Datenausgang (52) zum Senden von Betriebs- und Maschinendaten des Medizingeräts (500) an einen damit verbundenen Dateneingang (12) der Vorrichtung (100) und einen Spannungsausgang (51) zum Bereitstellen einer Betriebsspannung an einen damit verbundenen Spannungseingang (11) der Vorrichtung (100) zur Versorgung der Vorrichtung (100) umfasst.

10. Verfahren zum Übertragen von Betriebs- und Maschinendaten eines Medizingeräts (500), bevorzugt eines Dialysegeräts, an eine Auswertevorrichtung (60), das Verfahren aufweisend die folgenden Schritte:
- Bereitstellen einer Vorrichtung (100) umfassend einen Dateneingang (12), eine programmierbare Speichereinheit (20) einen Spannungseingang (11) und eine Übertragungseinheit (30);
- Verbinden der Vorrichtung (100) mit dem Medizingerät (500), derart dass die Vorrichtung (100) über den Spannungseingang (11) mit einer von dem Medizingerät (500) bereitgestellten Betriebsspannung versorgt wird;
- Empfangen von Betriebs- und Maschinendaten des Medizingeräts (500) über den Dateneingang (12);
- Speichern zumindest eines Teils der Betriebs- und Maschinendaten in der Speichereinheit (20);
- Übertragen der gespeicherten Betriebs- und Maschinendaten an die Auswertevorrichtung (60) mittels der Übertragungseinheit (30)
wobei die in der Speichereinheit (20) gespeicherten Betriebs- und Maschinendaten drahtlos an die Auswertevorrichtung (60) übertragen werden während die Vorrichtung (100) mit dem Medizingerät (500) verbunden ist und
wobei die Vorrichtung die Kommunikation nach außen auf Senden beschränkt oder unterbindet, wenn ein dem Betriebszustand des Medizingeräts entsprechendes Signal von dem Medizingerät übertragen wird.

11. Verfahren gemäß Anspruch 10, wobei das Signal ein(en) den Betriebszustand beschreibendes Datum und/oder Zeitstempel umfasst und/oder wobei das Signal ein Vorliegen eines aktuellen Behandlungsmodus beschreibt.

12. Verfahren nach Anspruch 10 oder 11, ferner aufweisend die folgenden Schritte:
- Verbinden der Vorrichtung (100) mit der Auswertevorrichtung (60) und
- Übertragen einer Konfigurationsvorgabe von der Auswertevorrichtung (60) an die programmierbare Speichereinheit (20).

13. Verfahren nach einem der Ansprüche 10 bis 12, wobei das Übertragen der Konfigurationsvorgabe von der Auswertevorrichtung (60) durchgeführt wird, während die Vorrichtung (100) mit dem Medizingerät (500) verbunden ist.

14. Verfahren nach einem der Ansprüche 10 bis 13, wobei das Verbinden der Vorrichtung (100) mit dem Medizingerät (500) und/oder ein Trennen der Vorrichtung (100) von dem Medizingerät (500) während des Betriebs des Medizingeräts (500) durchgeführt wird.

## Claims

1. A device (100) for transmitting operating and machine data of a medical apparatus (500), preferably a dialysis apparatus, to an evaluation device (60), wherein the device (100) comprises
- a data input (12) for receiving operating and machine data of the medical apparatus (500),
- a programmable memory unit (20), which is configured to store at least part of the received operating and machine data,
- a voltage input (11) which is configured to supply the device (100) with an operating voltage, provided by the medical apparatus (500), and
- a transmission unit (30) which is configured to transmit the stored operating and machine data to the evaluation device (60),
wherein the transmission unit (30) is configured to wirelessly transmit the operating and machine data, stored in the storage unit (20), to the evaluation device (60) while the device (100) is connected to the medical apparatus (500), and
wherein the device (100) is configured to prevent external communication or limit it to transmission when a signal, corresponding to the operating state of the medical apparatus (500), is transmitted from the medical apparatus.

2. The device (100) according to claim 1, wherein the signal comprises a date and/or a time stamp describing the operating state and/or wherein the signal describes a presence of a current treatment mode.

3. The device (100) according to claim 1 or 2, wherein
- the transmission unit (30) is configured to receive a configuration specification from the evaluation device (60) and to forward it to the programmable memory unit (20) and
- the programmable memory unit (20) is configured by the configuration specification to store operating and machine data in accordance with the configuration specification.

4. The device (100) according to the preceding claim, wherein receiving the configuration specification by the transmission unit (30) is blocked during operation of the medical apparatus (500).

5. The device (100) according to any one of the preceding claims, wherein the device (100) is configured to be connected to and/or disconnected from the medical apparatus (500) during operation of the medical apparatus (500).

6. The device (100) according to any one of the preceding claims, wherein the storage unit (20) has a processing unit for processing at least part of the operating and machine data.

7. The device (100) according to any one of the preceding claims, wherein the device (100) is configured in the manner of a dongle.

8. The device (100) according to any one of the preceding claims, comprising a housing (40) configured to be electrically insulating which contains the storage unit (20) and the transmission unit (30).

9. A system comprising a device (100) according to any one of the preceding claims and a medical apparatus (500), preferably for carrying out a dialysis treatment, wherein the device (100) has a first connection component (10) and the medical apparatus (500) has a second connection component (50), and the device (100) is inserted into and connected to the second connection component (50) of the medical apparatus (500) by means of the first connection component (10), and wherein the medical apparatus (500) comprises a data output (52) for sending operating and machine data of the medical apparatus (500) to a data input (12) of the device (100) connected thereto and a voltage output (51) for providing an operating voltage to a voltage input (11) of the device (100) connected thereto for supplying the device (100).

10. A method for transmitting operating and machine data of a medical apparatus (500), preferably a dialysis machine, to an evaluation device (60), the method having the following steps:
- providing a device (100) comprising a data input (12), a programmable memory unit (20), a voltage input (11) and a transmission unit (30);
- connecting the device (100) to the medical apparatus (500) such that the device (100) is supplied, via the voltage input (11), with an operating voltage, provided by the medical apparatus (500);
- receiving operating and machine data of the medical apparatus (500) via the data input (12);
- storing at least part of the operating and machine data in the storage unit (20);
- transmitting the stored operating and machine data to the evaluation device (60) by means of the transmission unit (30)
wherein the operating and machine data, stored in the storage unit (20), are transmitted wirelessly to the evaluation device (60) while the device (100) is connected to the medical apparatus (500) and
wherein the device prevents external communication or limits it to transmission when a signal, corresponding to the operating state of the medical apparatus, is transmitted from the medical apparatus.

11. The method according to claim 10, wherein the signal comprises a date and/or a time stamp describing the operating state and/or wherein the signal describes a presence of a current treatment mode.

12. The method according to claim 10 or 11, further having the following steps:
- connecting the device (100) to the evaluation device (60) and
- transmitting a configuration specification from the evaluation device (60) to the programmable memory unit (20).

13. The method according to any one of claims 10 to 12, wherein the transmitting of the configuration specification is carried out by the evaluation device (60) while the device (100) is connected to the medical apparatus (500).

14. The method according to any one of claims 10 to 13, wherein connecting the device (100) to the medical apparatus (500) and/or disconnecting the device (100) from the medical apparatus (500) is carried out during operation of the medical apparatus (500).

## Revendications

1. Dispositif (100) pour transmettre des données de fonctionnement et des données de machine d'un appareil médical (500), de préférence d'un appareil de dialyse, à un dispositif d'évaluation (60), dans lequel le dispositif (100) comprend
- une entrée de données (12), pour recevoir des données de fonctionnement et des données de machine de l'appareil médical (500),
- une unité de mémoire (20) programmable, qui est configurée pour stocker au moins une partie des données de fonctionnement et des données de machine reçues,
- une entrée de tension (11), qui est configurée pour alimenter le dispositif (100) en tension de fonctionnement fournie par l'appareil médical (500), et
- une unité de transmission (30), qui est configurée pour transmettre les données de fonctionnement et les données de machine stockées au dispositif d'évaluation (60),
dans lequel l'unité de transmission (30) est configurée pour transmettre sans fil au dispositif d'évaluation (60) les données de fonctionnement et les données de machine stockées dans l'unité de mémoire (20) pendant que le dispositif (100) est connecté à l'appareil médical (500), et
dans lequel le dispositif (100) est configuré pour limiter ou pour empêcher la communication vers l'extérieur à l'émission lorsqu'un signal correspondant à l'état de fonctionnement de l'appareil médical (500) est transmis par l'appareil médical.

2. Dispositif (100) selon la revendication 1, dans lequel le signal comprend un(e) date et/ou horodatage décrivant l'état de fonctionnement et/ou dans lequel le signal décrit la présence d'un mode de traitement actuel.

3. Dispositif (100) selon la revendication 1 ou la revendication 2, dans lequel
- l'unité de transmission (30) est configurée pour recevoir une consigne de configuration du dispositif d'évaluation (60) et pour la transférer à l'unité de mémoire (20) programmable et
- l'unité de mémoire (20) programmable est configurée par la consigne de configuration pour stocker des données de fonctionnement et de machine conformément à la consigne de configuration.

4. Dispositif (100) selon la revendication précédente, dans lequel la réception de la consigne de configuration par l'unité de transmission (30) est bloquée pendant le fonctionnement de l'appareil médical (500).

5. Dispositif (100) selon l'une quelconque des revendications précédentes, dans lequel le dispositif (100) est configuré pour être connecté à l'appareil médical (500) et/ou déconnecté de celui-ci lors du fonctionnement de l'appareil médical (500).

6. Dispositif (100) selon l'une quelconque des revendications précédentes, dans lequel l'unité de mémoire (20) présente une unité de traitement pour traiter au moins une partie des données de fonctionnement et de machine.

7. Dispositif (100) selon l'une quelconque des revendications précédentes, dans lequel le dispositif (100) est réalisé à la façon d'un dongle.

8. Dispositif (100) selon l'une quelconque des revendications précédentes, comprenant un boîtier (40) réalisé de manière électriquement isolante qui contient l'unité de mémoire (20) et l'unité de transmission (30).

9. Système comprenant un dispositif (100) selon l'une des revendications précédentes et un appareil médical (500), de préférence pour effectuer un traitement par dialyse, dans lequel le dispositif (100) présente un premier composant de connexion (10) et l'appareil médical (500) présente un deuxième composant de connexion (50), et le dispositif (100) est inséré dans le deuxième composant de connexion (50) de l'appareil médical (500) et connecté à celui-ci au moyen du premier composant de connexion (10), et dans lequel l'appareil médical (500) comprend une sortie de données (52) pour envoyer des données de fonctionnement et des données de machine de l'appareil médical (500) à une entrée de données (12) du dispositif (100) connectée à celui-ci et une sortie de tension (51) pour fournir une tension de fonctionnement à une entrée de tension (11) du dispositif (100) connectée à celui-ci pour alimenter le dispositif (100).

10. Procédé pour transmettre des données de fonctionnement et des données de machine d'un appareil médical (500), de préférence d'un appareil de dialyse, à un dispositif d'évaluation (60), le procédé présentant les étapes suivantes :
- la fourniture d'un dispositif (100) comprenant une entrée de données (12), une unité de mémoire (20) programmable, une entrée de tension (11) et une unité de transmission (30) ;
- la connexion du dispositif (100) à l'appareil médical (500) de telle sorte que le dispositif (100) soit alimenté par l'entrée de tension (11) en tension de fonctionnement fournie par l'appareil médical (500) ;
- la réception des données de fonctionnement et des données de machine de l'appareil médical (500) via l'entrée de données (12) ;
- le stockage d'au moins une partie des données de fonctionnement et des données de machine dans l'unité de mémoire (20) ;
- la transmission, au moyen de l'unité de transmission (30), des données de fonctionnement et des données de machine stockées au dispositif d'évaluation (60),
dans lequel les données de fonctionnement et de machine stockées dans l'unité de mémoire (20) sont transmises sans fil au dispositif d'évaluation (60) pendant que le dispositif (100) est connecté à l'appareil médical (500), et
dans lequel le dispositif limite ou empêche la communication vers l'extérieur à l'émission lorsqu'un signal correspondant à l'état de fonctionnement de l'appareil médical est transmis par l'appareil médical.

11. Procédé selon la revendication 10, dans lequel le signal comprend un(e) date et/ou horodatage décrivant l'état de fonctionnement et/ou dans lequel le signal décrit la présence d'un mode de traitement actuel.

12. Procédé selon la revendication 10 ou la revendication 11, présentant en outre les étapes suivantes :
- la connexion du dispositif (100) au dispositif d'évaluation (60) et
- la transmission d'une consigne de configuration du dispositif d'évaluation (60) à l'unité de mémoire (20) programmable.

13. Procédé selon l'une quelconque des revendications 10 à 12, dans lequel la transmission de la consigne de configuration est effectuée par le dispositif d'évaluation (60) pendant que le dispositif (100) est connecté à l'appareil médical (500).

14. Procédé selon l'une quelconque des revendications 10 à 13, dans lequel la connexion du dispositif (100) à l'appareil médical (500) et/ou une déconnexion du dispositif (100) de l'appareil médical (500) est effectuée pendant le fonctionnement de l'appareil médical (500).
